# EUROPEAN PATENT APPLICATION

(11) **EP 4 516 286 A1**
(43) Date of publication of application: **05.03.2025**
(21) Application number: 23194612.0
(22) Date of filing: 31.08.2023
(51) Int. Cl.: A61K 9/00, A61K 38/47, A61K 47/26, C12N 15/86

(54) **TREHALOSE-BASED COMBINED THERAPY FOR LYSOSOMAL DAMAGE**

(71) Applicant: GENETHON, 91000 Evry-Courcouronnes (FR); Institut National de la Santé et de la Recherche Médicale, 75013 Paris (FR); Université d'Evry Val d' Essonne, 91000 Evry-Courcouronnes (FR)
(72) Inventor: Israeli, David, 75012 Paris (FR); Jaber, Abbass, 94700 Maisons-Alfort (FR)
(74) Representative: Axe PI

(57) **Abstract**

The present invention relates to a composition comprising at least a carbohydrate for use in the prevention and/or treatment of lysosomal damage. It also relates to a combination of said composition with gene therapy.

## Description

### FIELD OF THE INVENTION

The present invention relates to a composition comprising at least one carbohydrate combined with gene therapy for its use in the prevention and/or the treatment of lysosomal damage, preferably for use in the prevention and/or treatment of hereditary myopathy.

### BACKGROUND

Lysosome is a monolayer vesicle rich in acidic hydrolases. It was previously found to be an extremely important intracellular degradation site, serving as the terminal destination during phagocytosis and autophagy. It has been demonstrated that lysosomes are capable of sensing and addressing cellular signals and playing essential roles in coping with stress stimuli. For example, lysosomes are programmed to monitor the intracellular levels of nutrients and energy, thus engaging in nutrient sensing, metabolic regulation, and cellular homeostatic maintenance by providing an initial platform for the activation of mammalian target of rapamycin complex 1 (mTORC1) and adenosine 5'-monophosphate (AMP)-activated protein kinase (AMPK). Damage to lysosomes not only disrupts cellular clearance ability but also results in the leakage of multiple enzymes, triggering the activation of a series of cell death pathways. Lysosomal damage is involved in various forms of cell death, such as apoptosis, necroptosis, pyroptosis, ferroptosis and lysoptosis, hinting that it is of great significance to the clarification of the potential mechanism of lysosomal damage and further exploration of efficient treatments based on lysosomal quality control (LQC). Indeed, the structural and functional abnormalities of lysosomes are critical for the pathogenesis and development of various diseases, including lysosomal storage diseases, cancer, neurodegenerative diseases, inflammatory diseases, cardiovascular diseases, and muscular pathologies, making LQC a novel pivotal target for treatments. Endolysosome damage consists of a series of specific cellular responses to lysosomal damage, including lysosomal repair, lysophagy, and lysosomal regeneration, which have been identified as being crucial for maintaining intracellular homeostasis.

A study showed that dysregulated lysosomal pH disrupts muscle homeostasis and causes muscle degeneration, e.g., in muscular dystrophies like Duchenne muscular dystrophy (DMD). (Coffey, Elizabeth C et al. "Lysosomal Function Impacts the Skeletal Muscle Extracellular Matrix." Journal of developmental biology vol. 9,4 52. 23 Nov. 2021, doi: 1 0.3390/jd b9040052).

Multiple therapies in DMD were demonstrated in preclinical models and in clinical trials. While gene therapy for DMD seems to be promising in animal preclinical data, the ongoing translation clinical trials produced only a modest success.

Furthermore, none of these trials target the lysosome.

Recent progress in therapeutic approaches has allowed new strategies to be investigated, including pharmacological, gene-based, and cell-based therapies. Gene and cell-based therapies are still limited by poor targeting and low efficiency in fibrotic dystrophic muscle; therefore, it is increasingly evident that future treatments will have to include "combined therapies" to reach maximal efficiency.

Hence, there remains a need for the development of a treatment that would help to get better results when it gets combined with gene therapy in pathologies that are associated with lysosomal damage.

### PROBLEM TO BE SOLVED

The technical problem underlying the present invention is notably to provide a treatment that specifically targets lysosomal damage and that will be beneficial when it is combined with gene therapy.

Another major problem is the therapeutic window between efficacy and toxicity. A very high viral dose is required for intravenous or intramuscular application in gene therapy. Toxicity and in particular cardiotoxicity are very serious risk. Potentiating therapeutic efficacy shall allow to reduce viral dose and the associated risk.

The technical problem is solved by the embodiments provided herein and as characterized in the appended claims.

### SUMMARY OF THE INVENTION

The invention relates to a composition comprising at least one carbohydrate for use in the prevention and/or treatment of lysosomal damage, wherein said composition is combined with gene therapy.

The Applicant has been able to demonstrate that in muscular pathologies, a lysosomal damage is only partially corrected by gene therapy. Co-administration of selected carbohydrates may improve lysosomal correction and the clinical benefit of treated patients.

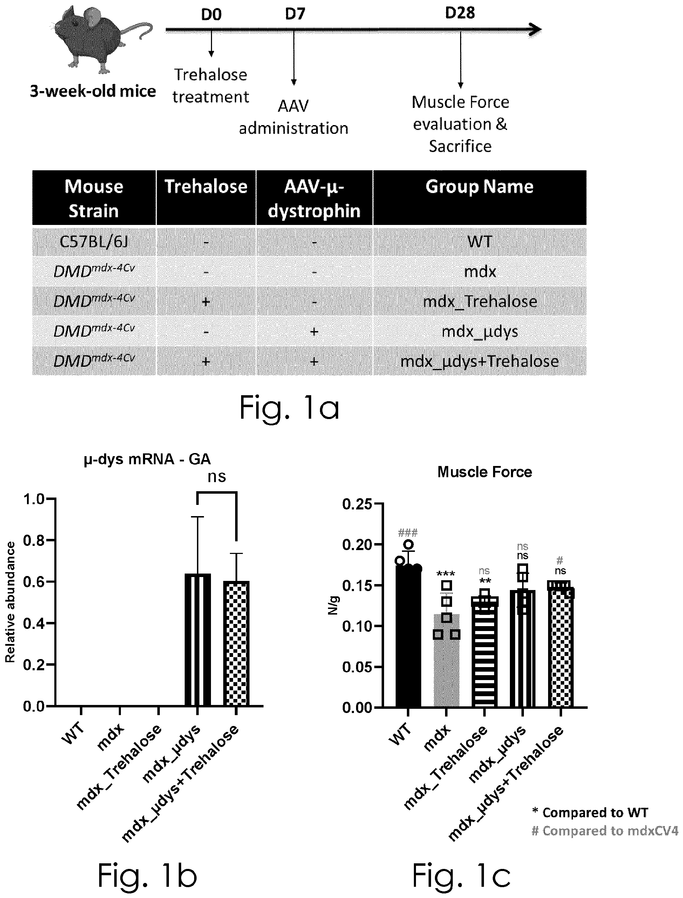

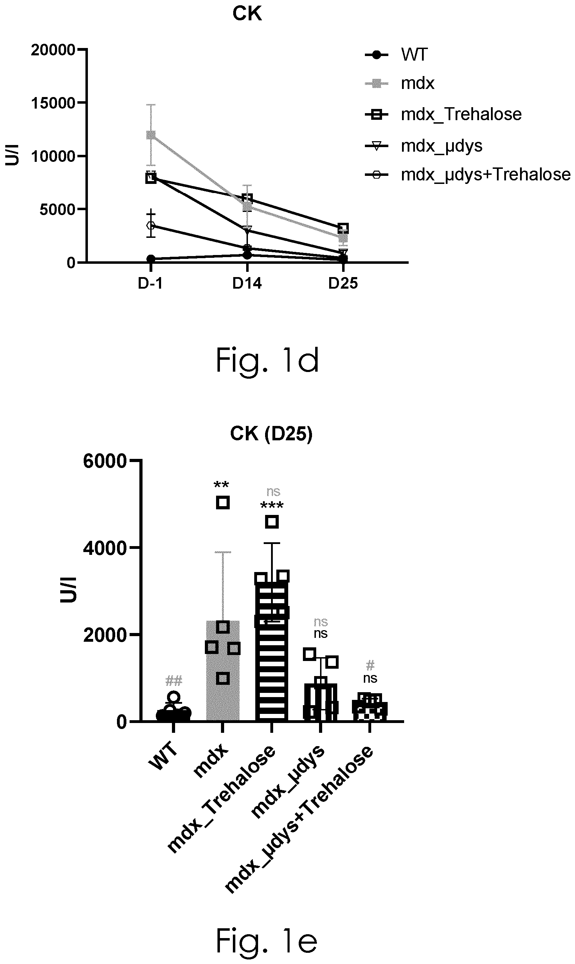

Further aspects and advantages of the present invention are described in the following description (with reference to Figures 1 to 3), which should be regarded as illustrative and not limiting the scope of the present application.

### BRIEF DESCRIPTION OF THE DRAWINGS

**Figure 1** shows experiment outline. 3-week-old Mdx-4Cv mice were treated with Trehalose, AAV-µdystrophin or a combination between Trehalose and µ-dystrophin (**1a**). No difference of expression of µ-dystrophin was detected at the mRNA level between AAV-µdystrophin treated mice and combined therapy group (**2b**). Muscle force was evaluated with an escape test. Mdx mice had significantly less muscle force compared to WT mice. A significant increase of muscle force compared to untreated mdx was detected only with combined therapy treated mice (**2c**). (*1-way ANOVA with Tukey multiple comparisons,* ^{∗}*(black)*= *compared to WT,* # *(gray)* = *compared to mdx,* ^{∗}<*0.05,* ^{∗∗}<*0.01,* ^{∗∗∗}<*0.001,* ^{∗∗∗∗}<*0.0001)*.
   Graphs **1d** and **1e** show evaluation of circulating biomarker creatine kinase (CK). At day 25 of treatment, a decrease of CK is noticeable for µ-dystrophin treated mice, albeit not significantly different from untreated mdx mice. Only mice treated with both trehalose and µ-dystrophin had a significantly lower CK level compared to untreated mdx mice. (*1-way ANOVA with Tukey multiple comparisons,* ^{∗}<*0.05*, ^{∗∗}<*0.01,* ^{∗∗∗}<*0.001*, ^{∗∗∗∗}<*0.0001*)

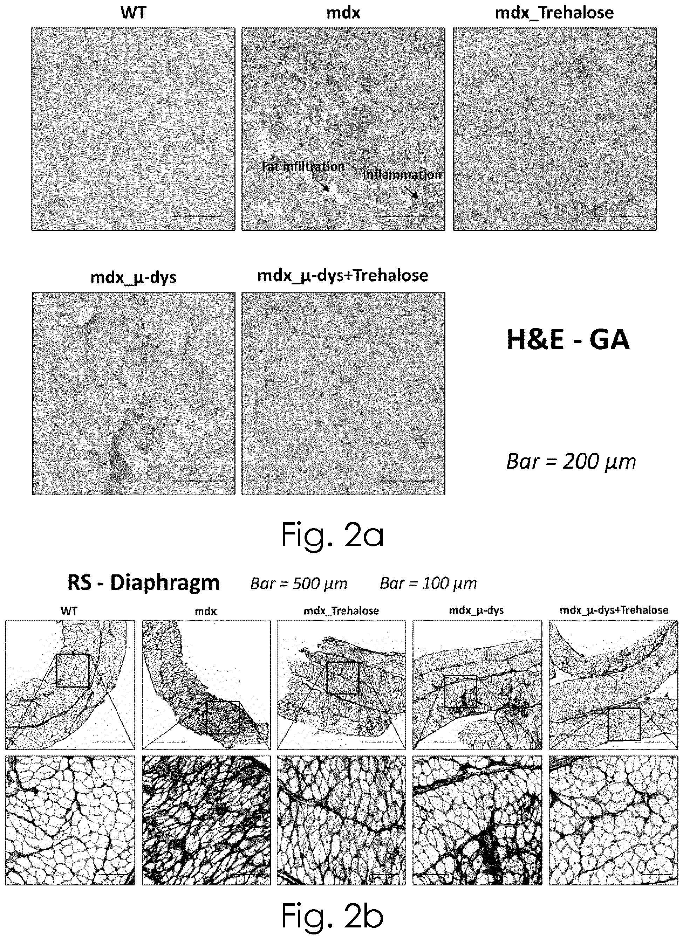

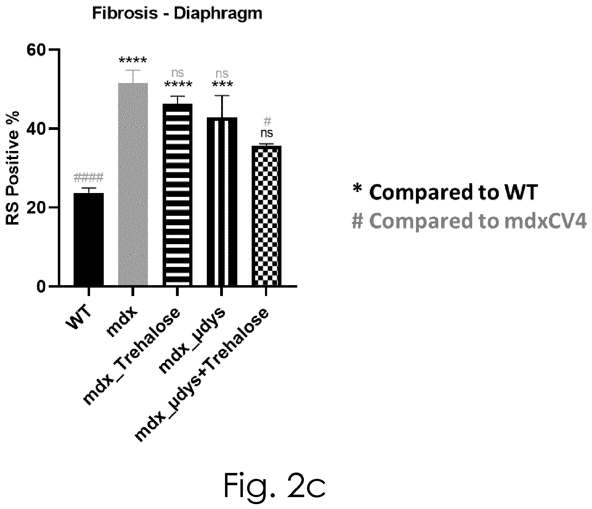

**Figure 2** illustrates histological evaluation of treated mice. H&E staining of Gastrocnemius muscle (GA) (**1a**) show hallmark characteristics of dystrophic muscle in mdx mice, including fat infiltration, deposit of inflammatory cells, centronucleated myofibers and heterogeneous fiber diameters. Some of these parameters were improved with Trehalose only treatment (less apparent inflammation and fat infiltration) or µ-dystrophin. interestingly, combined therapy group showed complete restoration of muscle histology to healthy state, with homogenous distribution of myofibers. (bar = *200µm*).
   Red Sirius staining (**2b**) show fibrosis on transversals sections of diaphragm muscle. Black deposition between myofibers represents collagen accumulation in dystrophic muscle. (*Big bar* = *500µm, small bar* = *100 µm*). Quantification of fibrotic area in transversal sections (**2c**) show significantly higher fibrosis of mdx muscle compared to WT. Treatment with trehalose or µ-dystrophin alone did not reduce fibrosis significantly. Interestingly, combined treatment of µ-dystrophin and trehalose reduced fibrosis close to the WT level (non-significant difference from WT, significantly lower than mdx). (*1-way ANOVA with Tukey multiple comparisons*, ^{∗}<*0.05*, ^{∗∗}<*0.01,* ^{∗∗∗}<*0.001,* ^{∗∗∗∗}<*0.0001*)

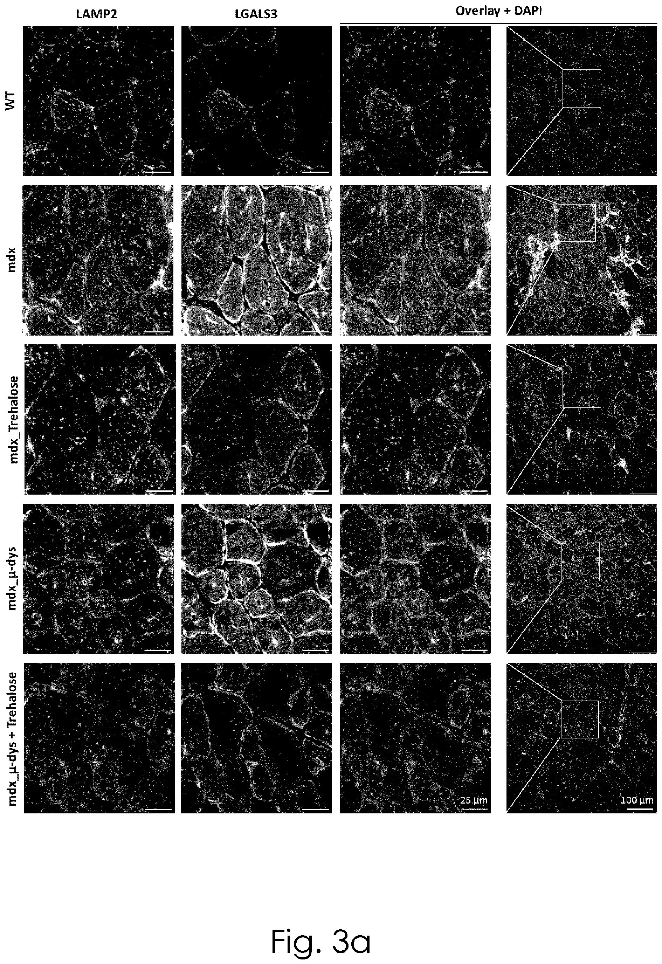

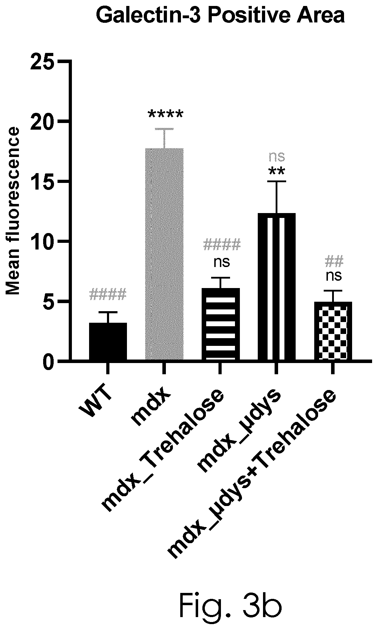

**Figure 3a** illustrates immunodetection of LAMP2 and LGALS3 (galectin 3) in the TA muscle of WT, mdx and treated mice. In the left column of figure 3a, LAMP2 that specifically stains the lysosome, is increasingly expressed in the TA muscle of the mdx mouse compared to the WT mouse, which indicates lysosomal perturbation and enlargement in the mdx muscle. In the middle column, the "Puncta" pattern of LGALS3 stain, is increasingly visible in the mdx muscle compared to the muscle of the healthy control mouse. In the two right columns, colocalization of LGALS3 and the lysosome marker LAMP2 is visible in the mdx muscle, indicating increased LGALS3 expression in the lysosome. The puncta pattern of LGALS3/LAMP2 is remarkably decreased with trehalose treatment to WT level, but not with µ-dystrophin only treatment. Combined treatment of µ-dystrophin and trehalose and µ-dystrophin showed similar pattern to WT muscle, with almost no puncta detected inside the myofibers. **Figure 3b** show quantification of LGALS3 positive area on transversal sections of TA muscles from different mice. mdx muscle had a significantly higher positive LGALS3 area compared to WT, which was restored to WT level for trehalose treated mice (significantly lower than mdx, non-different from WT) but not for -dystrophin only treated mice. Mice treated with both µ-dystrophin and trehalose had also a non-significantly different LGALS3 positive area from WT mice. (*1-way ANOVA with Tukey multiple comparisons,* ^{∗}<*0.05,* ^{∗∗}<*0.01,* ^{∗∗∗}<*0.001, ^{∗∗∗∗}*<*0.0001*)

### DETAILED DESCRIPTION

The present invention provides a composition comprising at least one carbohydrate for use in the prevention and/or treatment of lysosomal damage.

The term "composition" as used herein preferably refers to a pharmaceutical composition that contains pharmaceutical acceptable medium.

A pharmaceutically acceptable medium includes any, and all solvents, dispersion media, coatings, antibacterial and antifungal agents, isotonic and absorption delaying agents and the like suitable for administration to a mammalian host. The use of such media and agents for pharmaceutical active substances is well known in the art. Supplementary active ingredients can also be incorporated into the medicament of the present invention.

The term "carbohydrate" as used herein refers to a biomolecule consisting of carbon, hydrogen, and oxygen atoms. According to the present invention, carbohydrate means saccharide, which is a group that includes sugars, starch, and cellulose. The saccharides are divided into four chemical groups: monosaccharides, disaccharides, oligosaccharides, and polysaccharides. Monosaccharides and disaccharides, the smallest (lower molecular weight) carbohydrates, are commonly referred to as sugars. While the scientific nomenclature of carbohydrates is complex, the names of the monosaccharides and disaccharides very often end in the suffix -ose, which was originally taken from the word glucose, and is used for almost all sugars.

In a preferred embodiment, the carbohydrate added to the composition is a disaccharide. A disaccharide, also called a double sugar or biose, is the sugar formed when two monosaccharides are joined by glycosidic linkage. Like monosaccharides, disaccharides are simple sugars soluble in water. There are two functionally different classes of disaccharides:
- Reducing disaccharides, in which one monosaccharide, the reducing sugar of the pair, still has a free hemiacetal unit that can perform as a reducing aldehyde group; lactose, maltose and cellobiose are examples of reducing disaccharides, each with one hemiacetal unit, the other occupied by the glycosidic bond, which prevents it from acting as a reducing agent. They can easily be detected by the Woehlk test or Fearon's test on methylamine.
- Non-reducing disaccharides, in which the component monosaccharides bond through an acetal linkage between their anomeric centers. This results in neither monosaccharide being left with a hemiacetal unit that is free to act as a reducing agent. Sucrose and trehalose are examples of non-reducing disaccharides because their glycosidic bond is between their respective hemiacetal carbon atoms. The reduced chemical reactivity of the non-reducing sugars, in comparison to reducing sugars, may be an advantage where stability in storage is important.

The carbohydrate of the present invention is preferably used as an activator of lysosomal biogenesis.

In a more preferred embodiment, the carbohydrate is selected from trehalose, lactulose and/or melibiose.

Trehalose is a nonreducing sugar commonly found in bacteria, fungi, yeast, insects, and plants, consisting of two molecules of glucose. It has strong hydrophilic properties and resistance to acidic conditions and to enzymatic cleavage. The ability to interact spatially with polar groups of biomolecules provides trehalose with protein-protective properties. It prevents the denaturation of proteins during drying, preserves therapeutic antibodies, protects yeast from thermal denaturation, and prevents protein aggregation. It is a natural cytoprotective agent that prevents damage to invertebrates during fluctuations of environmental conditions. The cytoprotective properties of trehalose associated with the protection of spatial structure of proteins from adverse factors and the use in cell bio-conservation have long been known. Trehalose has been widely employed in the cryopreservation of cells because it has cryoprotective properties. It is applied to cryopreservation of spermatozoa, hepatocytes, kidney cells, stem cells and other cells.

It is believed that trehalose is not synthesized in the body of vertebrates because genes for the biosynthesis of this disaccharide have not been found. Nonetheless, its presence has been noted in the cells of the intestines, liver, kidneys, and mammalian brain (particularly in the hippocampus and the cortex); this presence is related to the consumption of plant foods and of fungi containing trehalose. It was noted that trehalose is consumed in the brain exclusively by neurons, where it is necessary for branching of neurites.

Some bacteria, fungi, plants, and invertebrate animals synthesize it as a source of energy, and to survive freezing and lack of water. In mammals, trehalose is usually ingested with food.

Trehalose has a therapeutic effect in various experimental models of diseases, especially those involving brain damage. The beneficial effect of trehalose on models of various diseases such as diabetes, liver steatosis, infections, cancer, and neurodegenerative diseases has already been analyzed previously. Now, it is clear that the range of trehalose therapeutic targets may be vast. At least several mechanisms are known for the possible therapeutic effect of trehalose, e.g., autophagy induction, chaperone-like activity, anti-inflammatory activity, and promotion of antioxidant defense. In addition, trehalose is an FDA approved drug characterized by extremely low toxicity which facilitates its therapeutic use.

Lately, trehalose has been undergoing clinical trials for the treatment of various pathologies, including neuropathologies, such as bipolar depression (NCT02800161), Parkinson disease (NTC05355064) and Alzheimer (NTC05332678).

The precise therapeutic mechanism of trehalose is not completely understood, yet one of the propositions is the acceleration of lysosomal biogenesis, which is associated also by accelerated autophagy (Pupyshev, Alexander B et al. "Disaccharide trehalose in experimental therapies for neurodegenerative disorders: Molecular targets and translational potential." Pharmacological research vol. 183 (2022): 106373. doi: 10.10 16/j.phrs.2022.1 06373).

Lactulose is a synthetic disaccharide formed from one molecule each of the monosaccharides fructose and galactose. Lactulose is not normally present in raw milk but is a product of heat processes: the greater the heat, the greater amount of this substance. It is produced commercially by isomerization of lactose.

Lactulose is not absorbed in the small intestine nor broken down by human enzymes, thus stays in the digestive bolus through most of its course, causing retention of water through osmosis leading to softer, easier-to-pass stool. It has a secondary laxative effect in the colon, where it is fermented by the gut flora, producing metabolites which have osmotic powers and peristalsis-stimulating effects (such as acetate), but also methane associated with flatulence. It is used in the treatment of constipation and hepatic encephalopathy. It has also been used in the diagnosis of gastrointestinal disorders.

Melibiose is a reducing disaccharide formed by an a-1,6 linkage between galactose and glucose. It differs from lactose in the chirality of the carbon where the galactose ring is closed, and that the galactose is linked to a different point on the glucose moiety. It can be formed by invertase-mediated hydrolysis of raffinose, which produces melibiose and fructose. Melibiose can be broken down into its component saccharides, glucose, and galactose, by the enzyme alpha-galactosidase, such as MEL1 from Saccharomyces *pastorianus.*

In the present invention, the carbohydrate is preferably trehalose.

The term "treatment", and related terms, such as "treating" and "treat" as used herein, relates to an amelioration, prophylaxis or reversal of a disease or disorder, or at least one discernible symptom thereof. It also refers to an improvement, prophylaxis, or reversal of at least one measurable physical parameter related to the disease or disorder being treated, which is not necessarily perceptible to the subject. In another embodiment, the term "treatment" refers to the inhibition or slowing down of the progression of a disease or disorder, either physically, for example, the stabilization of a discernible symptom, physiologically, for example, the stabilization of a physical parameter, or both. The term "treatment" also refers to delaying the onset of a disease or disorder. In some particular embodiments, the compositions of interest are administered as a preventive measure.

In this context, the term "prevention" refers to a reduction in the risk of acquiring a specified disease or disorder.

The term "lysosomal damage" as used herein refers to lysosome dysfunction and lysosomal stress.

Lysosomes are single membrane acidic organelles that function to degrade intracellular cargo. These organelles are heterogeneous in size (range 0.5 to 1.5 nm), have intraluminal pH values ranging from 4.5 to 6.0, and contain over 60 different acidic hydrolase enzymes. The intraluminal pH of lysosomes is maintained by membrane-resident ion channels and vacuolar-ATPases. The acidic lumen of lysosomes provides an environment that is conducive for optimal activity levels of resident hydrolase enzymes which catalyze the degradation of cargo that is received via endocytic or autophagic pathways (Kornfeld and Mellman, 1989). Lysosomes also contain readily releasable stores of biologically important divalent cations including iron and calcium. Intracellularly, the positioning of endolysosomes is dynamic and their positioning in the cytoplasm changes markedly according to many factors including cell type and intraluminal pH; highly acidic endolysosomes are perinuclearly positioned while de-acidified endolysosomes are re-positioned more towards the periphery of cells near plasma membranes.

Lysosomes can receive cargo from endocytic or autophagic pathways. Once cargo enters the endolysosomal system it has two fates, it can either be trafficked toward lysosomes to be degraded or it can be recycled to the plasma membrane. Along the maturation path of endosomes to lysosomes, intraluminal vesicles begin to form in endosomes thus creating MVBs. Cargo within MVBs can be trafficked toward the plasma membrane to release contents into the extracellular space; these secreted ILVs are termed exosomes; a type of extracellular vesicles. The release of exosomes can potentially serve as intercellular signals, therapeutic delivery-vesicles, and biomarkers for various pathologies.

Autophagy is the means by which cytoplasmic material is degraded. There are different forms of autophagy such as macro-autophagy, micro-autophagy (selective autophagy), and chaperone-mediated autophagy. Intracellular cargo is engulfed by double membrane vesicles called autophagosomes; autophagosome formation, elongation, and maturation have been reviewed elsewhere. Important to the clearance of damaged proteins and dysfunctional organelles via autophagy is the fusion of autophagosomes with lysosomes forming autolysosomes. In post-mitotic cells, such as neurons, the autophagic-lysosome system is important for the maintenance of neuronal health.

Apart from their degradative roles, lysosomes also participate in intracellular nutrient sensing. Lysosomes use mTOR and TFEB to sense and regulate intracellular nutrient conditions, and the roles of mTOR and TFEB in lysosome nutrient sensing and cellular function were reviewed extensively elsewhere. mTOR is part of a larger protein complex that interacts with lysosome membranes and senses cytosolic nutrient levels. During high nutrient conditions, mTOR is recruited to lysosome membranes where it is activated and initiates downstream effectors that promote cell growth and proliferation. Conversely, during low nutrient conditions mTOR is inactive, disassociates from lysosome membranes, and is no longer able to phosphorylate TFEB; dephosphorylated TFEB promotes the production of lysosome genes.

Central to lysosome dysfunction appears to be an increase in intraluminal pH - lysosome deacidification. Lysosome de-acidification causes many changes to the biology of cells including endolysosome positioning in the cytoplasm, endolysosome sizes and volumes, intraluminal and cytosolic levels of divalent cations, expression levels and activity levels of acid hydrolases, and the aggregation of various proteins. And, as we will expand on later, these changes are also central to the concept of Lysosomal stress response (LSR).

The intracellular distribution (positioning) of endolysosomes in the cytoplasm is affected by endolysosome de-acidification; an increase in endolysosome pH causes redistribution of lysosomes away from the nucleus and more toward plasma membranes. The cytosolic pH which is influenced by H+ ions originating from endolysosomes also affects endolysosome positioning; cytosolic acidification promotes the localization of lysosomes towards plasma membranes and cytosolic alkalinization leads to perinuclear localization of lysosomes. Although the biological significance of the pH-dependent repositioning of endolysosomes in cells is unclear, some have shown that the location of endolysosomes dictates their pH and not that their pH dictates the location (Johnson et al., 2016). Microtubules and motor proteins also contribute to the translocation and clustering of endolysosomes (Matteoni and Kreis, 1987; Cabukusta and Neefjes, 2018).

Morphologically, endolysosome de-acidification leads to increased endolysosome size and an accumulation of undigested intracellular cargo. Endolysosome de-acidification also leads to impaired fusion between autophagosomes and lysosomes, and such changes have been implicated in cell death.

Endolysosomes contain readily releasable stores of biologically important divalent cations including calcium, iron, copper, and zinc. De-acidification of endolysosomes induces the efflux of calcium and iron such that accumulations are measurable in cytosol, mitochondria, and ER. As a result of decreased levels of cations in endolysosomes, downstream events can occur including ER stress as well as mitochondrial redox catastrophe and bioenergetic crisis. (Lakpa, Koffi Let al. "Lysosomal Stress Response (LSR): Physiological Importance and Pathological Relevance." Journal of neuroimmune pharmacology : the official journal of the Society on Neurolmmune Pharmacology vol. 16,2 (2021): 219-237. doi:10.1007/s11481-021-09990-7).

The term "lysosomal stress" as used herein refers to any disturbance in lysosomal membrane integrity, enzyme function, or internal pH that leads to cell damage or even diseases.

The Applicant has previously been able to demonstrate that Galectin-3 (Gal-3) puncta assay can be used as a generic biomarker for the detection and the monitoring of lysosomal damage in neuromuscular diseases such as muscular dystrophies.

The galectin-3 puncta assay is based on the high binding affinity of the galectin-3 protein to glycan residues (a chemical particular form of sugar). An early symptom of the lysosomal stress and damage response is a chemico-structural change in the lysosome membrane. Under normal conditions, the particular forms of the glycan residue which are recognized by Galectin-3, are masked inside the hydrophobic environment of the lysosomal membrane, in a configuration that does not permit their recognition and binding by galectin-3. Therefore, under normal conditions, Galctin-3 expression in the myofiber in a cytoplasmic diffusible pattern. However, upon lysosomal stress and membrane damage, these glycan residues are exposed to the cytoplasmic hydrophilic cellular environment in a position that favors their binding by large number of galectin-3 molecules. The biological function of galectin-3 "sensing" of the damaged lysosomal membrane, is the activation and synchronization of the lysosomal stress response (PMID: 31813797). The detection of a puncta Galectin-3 patterns, by using the anti-galectin-3 antibody, is therefore a biological biomarker for the early-stage of the lysosomal stress and damage response.

According to the invention, the use of said composition is combined with gene therapy.

The terms "gene therapy" as used herein refer to a medical technology which aims to produce a therapeutic effect through the manipulation of gene expression or through altering the biological properties of living cells. More particularly, it " refers to the transfer of genetic material (e.g., DNA or RNA) of interest into a host to treat or prevent a genetic or acquired disease or condition. The genetic material of interest encodes a product (e.g., a polypeptide or functional RNA) whose production is desired in *vivo.* For example, the genetic material of interest can encode a hormone, receptor, enzyme, or polypeptide of therapeutic value. Alternatively, the genetic material of interest can encode a functional RNA of therapeutic value, such as an antisense RNA or a shRNA of therapeutic value. The concept of gene therapy is to fix a genetic problem at its source. If, for instance, in an (usually recessively) inherited disease a mutation in a certain gene results in the production of a dysfunctional protein, gene therapy could be used to deliver a copy of this gene that does not contain the deleterious mutation (named transgene), and thereby produces a functional protein. This strategy is referred to as gene replacement therapy.

A therapeutic transgene is selected and used to lead to a desired therapeutic outcome, in particular for achieving expression of said therapeutic transgene into a cell, tissue or organ into which expression of said therapeutic transgene is needed (i.e., target cell, tissue or organ). Therapy may be achieved by a number of ways, including by expressing a protein into a cell that does not express said protein, by expressing a protein into a cell that expresses a mutated version of the protein, by expressing an antisense RNA to induce gene repression or exon skipping, by expressing a silencing RNA such as a shRNA whose purpose is to suppress the expression of a protein, or by expressing a genome-editing enzyme whose purpose is to modify a target genomic sequence.

The gene is any nucleic acid sequence capable of modifying a target gene or target cellular pathway, in target cells, tissue or organ. For example, the gene may modify the expression, sequence or regulation of the target gene or cellular pathway. In some embodiments, the gene is a functional version of a gene or a fragment thereof. The functional version of said gene includes the wild-type gene, a variant gene such as variants belonging to the same family and others, or a truncated version, which preserves the functionality of the encoded protein at least partially. A functional version of a gene is useful for replacement or additive gene therapy to replace a gene, which is deficient or non-functional in a patient. In other embodiments, the gene is a gene which inactivates a dominant allele causing an autosomal dominant genetic disease. A fragment of a gene is useful as recombination template for use in combination with a genome editing enzyme.

The protein encoded by the transgene is any protein or peptide of interest such as with no limitations a protein encoded by a functional version of a non-functional or deficient gene for replacement or additive gene therapy; an antibody or antibody fragment, a genome-editing enzyme, or another protein.

The RNA encoded by the transgene of interest is advantageously complementary to a target DNA or RNA sequence or binds to a target protein. For example, the RNA is an interfering RNA such as a shRNA, a microRNA, a guide RNA (gRNA) for use in combination with a Cas enzyme or similar enzyme for genome editing, an antisense RNA capable of exon skipping such as a modified small nuclear RNA (snRNA) or a long non-coding RNA. The interfering RNA or microRNA may be used to regulate the expression of a target gene having altered expression in a target cell, tissue, or organ. The guide RNA in complex with a Cas enzyme or similar enzyme for genome editing may be used to modify the sequence of a target gene, in particular to correct the sequence of a mutated/deficient gene or to modify the expression of a target gene having altered expression in a target cell, tissue or organ. The antisense RNA capable of exon skipping is used in particular to correct a reading frame and restore expression of a deficient gene having a disrupted reading frame. In some embodiments, the RNA is a therapeutic RNA.

The genome-editing enzyme according to the invention is any enzyme or enzyme complex capable of modifying a target gene or target cellular pathway in target cells. For example, the genome-editing enzyme may modify the expression, sequence or regulation of the target gene or cellular pathway. The genome-editing enzyme is advantageously an engineered nuclease, such as with no limitations, a meganuclease, zinc finger nuclease (ZFN), transcription activator-like effector-based nuclease (TALENs), Cas enzyme from clustered regularly interspaced palindromic repeats (CRISPR)-Cas system and similar enzymes. The genome-editing enzyme, in particular an engineered nuclease such as Cas enzyme and similar enzymes, may be a functional nuclease which generates a double-strand break (DSB) or single-stranded DNA break (nickase such as Cas9(D10A) in the target genomic locus and is used for site-specific genome editing applications, including with no limitations: gene correction, gene replacement, gene knock-in, gene knock-out, mutagenesis, chromosome translocation, chromosome deletion, and the like. For site-specific genome editing applications, the genome-editing enzyme, in particular an engineered nuclease such as Cas enzyme and similar enzymes may be used in combination with a homologous recombination (HR) matrix or template (also named DNA donor template) which modifies the target genomic locus by double-strand break (DSB)-induced homologous recombination. In particular, the HR template may introduce a transgene of interest into the target genomic locus or repair a mutation in the target genomic locus, preferably in an abnormal or deficient gene having altered expression in target cells, tissue, or organ. The genome-editing enzyme, such as Cas enzyme and similar enzyme may be a DNA base-editor such as cytosine base-editor and adenine base-editor or a prime-editor. Base-editors can install all four transition mutations while Prime-editor expand the scope of donor-free precise DNA editing to not only all transition and transversion mutations, but small insertion and deletion mutations as well. Collectively, DNA base-editing and prime-editing tools enable precise nucleotide substitutions in a programmable manner, without requiring a donor template. Alternatively, the genome-editing enzyme, such as Cas enzyme and similar enzymes may be engineered to become nuclease-deficient and used as DNA-binding protein for various genome engineering applications in target cells, tissue, or organ, such as with no limitation: transcriptional activation, transcriptional repression, epigenome modification, genome imaging, DNA or RNA pull-down and the like.

The terms "combined with gene therapy" as used herein refer to both a therapy to correct the genetic defect and an additional one to address one of the secondary pathological features of the disease.

The composition according to the present invention combined with gene therapy is preferably used for the prevention and/or treatment of hereditary myopathy.

The terms "hereditary myopathy" as used herein refer to a heterogeneous group of inherited diseases primarily affecting the skeletal muscle tissue. These are caused by mutations in genes encoding proteins critical for muscle structure and function, with X-linked, autosomal-recessive or -dominant inheritance pattern. Hereditary myopathies include several forms of dystrophic and non-dystrophic disorders with a wide spectrum of genetic, biochemical, histological, and clinical features. A common characteristic is the presence of hypotonia and progressive or non-progressive muscle weakness. The onset of hereditary myopathies is commonly at birth, although they may become evident later in childhood or adulthood. Clinical severity is variable being the early-onset forms usually more severe. Diagnosis of hereditary muscle diseases involve physical and neurological evaluation, electromyography, and nerve conduction studies (EMG and NCS), magnetic resonance imaging, blood tests including creatine kinase levels (CK), which typically rises in muscle damage and histopathological makers in muscle biopsies. Advances in molecular genetics have allowed identifying an increasing number of genes linked to different forms of hereditary myopathies.

In a preferred embodiment, hereditary myopathy is selected from congenital myopathies, autophagic vacuolar myopathy and muscular dystrophy,
and wherein:
- autophagic vacuolar myopathy is preferably inclusion body myositis, VCP-related muscular dystrophy, type 2 glycogen storage disease with GAA mutation (pompé), and the toxic myopathies.
- muscular dystrophy is preferably DMD, myotonic dystrophy, Facioscapulohumeral muscular dystrophy (FSHD), Becker muscular dystrophy (BMD), distal Muscular Dystrophy, congenital muscular dystrophy, Emery-Dreifuss Muscular Dystrophy, Oculopharyngeal Muscular Dystrophy (OPMD), Limb girdle muscular dystrophies (LGMD) advantageously selected from Calpain-3-related limb-girdle muscular dystrophy R1 (LGMDR1), Dysferlin related limb girdle muscular dystrophy R2 (LGMDR2), Alpha sarcoglycan related limb girdle muscular dystrophy R3 (LGMDR3), Gamma sarcoglycan related limb girdle muscular dystrophy R5 (LGMDR5), FKRP related limb girdle muscular dystrophy R9 (LGMDR9), Dysferlin related limb girdle muscular dystrophy R2 type 2B (LGMD2B/R2), X-linked myotubular myopathy, BIN1-related centronuclear myopathy and DNM2-related centronuclear myopathy.

Congenital myopathies are genetic neuromuscular disorders characterized by typical histopathological alterations including type-1 fibers predominance and hypotrophy and presence of structural abnormalities such as rod-inclusions and cores, among others. Their clinical course is usually non-progressive or slowly progressive and their prognosis is mainly determined by the involvement of respiratory muscles. Unlike muscular dystrophies, patients with congenital myopathy typically exhibit normal or discretely increased levels of CK. The onset of the disease generally occurs in the neonatal period, and it has an estimated incidence of 1:25,000 live births. Clinically, congenital myopathies manifest with heterogeneous features such as generalized weakness, hypotonia, hyporeflexia, and poor muscle bulk. Congenital myopathies also presents with dysmorphic characteristics, secondary to the myopathy such as pectus carinatum (a chest malformation characterized by a protrusion of the sternum and ribs), scoliosis, joint-contractures, foot deformities, high-arched palate, and elongated facies. Different mutations in a same gene can cause different phenotypic forms of congenital myopathy, whereas mutations in different genes can induce muscle diseases with overlapping clinical and histological features, making difficult a specific diagnosis. However, based on the histological markers observed in muscle biopsies, congenital myopathies can be divided in five forms: nemaline myopathy, core myopathy, centronuclear myopathy, fiber-type disproportion myopathy, and myosin storage myopathy.

Autophagic vacuolar myopathy (AVM) consists of multiple rare genetic disorders with common histological and pathological features on muscle biopsy. The features highlighted are vacuolar membranes of the autophagic vacuoles having sarcolemmal characteristics and an excess of autophagic vacuoles. There are currently five types of AVM identified: Danon disease, X-linked myopathy with excessive autophagy (XMEA), Pompe Disease, infantile AVM, and adult-onset AVM with multiorgan involvement. The signs and symptoms become more severe over the course of the disease. It begins with an inability to pick up small objects and progresses to difficulty in walking. The age of onset varies from early childhood to late adulthood, affecting people of all ages. The disorders are caused by a mutation in different parts of the chromosome: XMEA is linked to mutations in the VMA21 gene at Xq28 while Danon disease and Pompe Disease are associated with LAMP2 gene located on the X chromosome and the GAA gene respectively. The cause of the disease is different mutation occurring in the aforementioned genes. For Danon disease, which is related to LAMP2, since the LAMP2 gene is responsible for the production of the LAMP-2 protein, which plays a role in the transport of cellular materials into the lysosome, mutations of the LAMP2 gene lead to little to no LAMP-2 protein production, impairing the transport of cellular materials into the lysosome. Without the LAMP-2 protein, fusion between autophagic vacuoles and lysosomes occurs much slower, leading to the accumulation of autophagic vacuoles. This accumulation leads to the breakdown of muscle cells, thereby causing the muscle weakness exhibited in Danon disease patients. Most of the AVMs are sex-linked, meaning the gene with regards to AVM is located on a sex chromosome.

Lysosomal myopathies are hereditary myopathies characterized morphologically by the presence of autophagic vacuoles. Autophagy plays an important role for the turnover of cellular components, particularly in response to starvation stimuli. In normal muscle, autolysosomes or autophagosomes are typically inconspicuous. In distinct neuromuscular disorders, however, lysosomes become structurally abnormal and functionally impaired, leading to the accumulation of autophagic vacuoles in myofibers. In some instances, the accumulation of autophagic vacuoles can be a prominent feature, implicating autophagy as a contributor to disease pathomechanism and/or progression. Important subclass of Lysosomal myopathies are the muscle glycogenoses, or glycogen storage diseases (which resulted due to perturbations of glycogen breakdown), included the Type 2 (Pompe disease), Type 3 (Cori disease), Type 4 (Andersen's disease), Type 5 (McArdle Disease), and Type 7 (Tarui disease). Another lysosomal myopathy is the Danon disease, due to mutations in the LAMP2 gene.

According to the present invention, said autophagic vacuolar myopathy is preferably inclusion body myositis or toxic myopathies.

Inclusion body myositis (IBM) is the most common inflammatory muscle disease in older adults. The disease is characterized by slowly progressive weakness and wasting of both proximal muscles (located on or close to the torso) and distal muscles (close to hands or feet), most apparent in the finger flexors and knee extensors. In IBM, two processes appear to occur in the muscles in parallel, one autoimmune and the other degenerative. Inflammation is evident from the invasion of muscle fibers by immune cells. Degeneration is characterized by the appearance of holes, deposits of abnormal proteins, and filamentous inclusions in the muscle fibers. sIBM is not inherited and is not passed on to the children of IBM patients. There are genetic features that do not directly cause IBM but that appear to predispose a person to getting IBM - having this particular combination of genes increases one's susceptibility to getting IBM. Some 67% of IBM patients have a particular combination of human leukocyte antigen genes in a section of the 8.1 ancestral haplotype in the center of the MHC class II region. sIBM is not passed on from generation to generation, although the susceptibility region of genes may be.

Toxic myopathies are described as the acute or subacute manifestation of muscle weakness, myalgia, hyperCKemia, or rhabdomyolysis that can occur in patients without an underlying known muscle disease when they are exposed to certain drugs. Muscle tissue is highly sensitive to many substances. Early recognition of toxic myopathies is important, as they potentially are reversible on removal of the offending drug or toxin, with greater likelihood of complete resolution the sooner this is achieved. Clinical features range from mild muscle pain and cramps to severe weakness with rhabdomyolysis, renal failure, and even death. The pathogenic bases can be multifactorial.

Muscular dystrophy (MD) is a group of inherited genetic conditions that gradually cause the muscles to weaken, leading to an increasing level of disability. MD is caused by changes (mutations) in the genes responsible for the structure and functioning of a person's muscles. The mutations are often inherited from a person's parents. They cause changes in the muscle fibers that interfere with the muscles' ability to function. Over time, this causes increasing disability. The muscle protein, dystrophin, is in most muscle cells and works to strengthen the muscle fibers and protect them from injury as muscles contract and relax. It links the muscle membrane to the thin muscular filaments within the cell. Dystrophin is an integral part of the muscular structure, an absence of dystrophin can cause impairments such as: healthy muscle tissue can be replaced by fibrous tissue and fat, causing inability to generate force. Respiratory and cardiac complications can occur as well. Although MD can affect several body tissues and organs, it most prominently affects the integrity of muscle fibers. Also, overall muscle strength and tendon reflexes are usually lessened or lost due to replacement of muscle by connective tissue and fat.

In an even more preferred embodiment, hereditary myopathy is muscular dystrophy chosen from DMD, myotonic dystrophy, Facioscapulohumeral muscular dystrophy (FSHD), Becker muscular dystrophy (BMD), distal Muscular Dystrophy, congenital muscular dystrophy, Emery-Dreifuss Muscular Dystrophy, Oculopharyngeal Muscular Dystrophy (OPMD), Limb girdle muscular dystrophies (LGMD) advantageously selected from Calpain-3-related limb-girdle muscular dystrophy R1 (LGMDR1), Dysferlin related limb girdle muscular dystrophy R2 (LGMDR2), Alpha sarcoglycan related limb girdle muscular dystrophy R3 (LGMDR3), Gamma sarcoglycan related limb girdle muscular dystrophy R5 (LGMDR5), FKRP related limb girdle muscular dystrophy R9 (LGMDR9), Dysferlin related limb girdle muscular dystrophy R2 type 2B (LGMD2B/R2).

Duchenne Muscular Dystrophy (DMD) is an X-linked recessive disorder. DMD symptom onset is in early childhood, usually between ages 2 and 3. The disease primarily affects boys, but in rare cases it can affect girls. It is characterized by muscle fiber wasting, functional impairment and eventual death due to respiratory and heart failure. The underlying causes are frame-shifting and nonsense mutations in the DMD gene, resulting in the absence of functional dystrophin protein. Intact dystrophin anchors the intracellular cytoskeleton to the extracellular matrix and thereby prevents membrane damage during muscle contraction.

Myotonic dystrophy is a type of muscular dystrophy. In myotonic dystrophy, muscles are often unable to relax after contraction. Other manifestations may include cataracts, intellectual disability, and heart conduction problems. In men, there may be early balding and an inability to father children. While myotonic dystrophy can occur at any age, onset is typically in the 20s and 30s. Myotonic dystrophy is caused by a genetic mutation in one of two genes. Mutation of the DMPK gene causes myotonic dystrophy type 1 (DM1). Mutation of CNBP gene causes type 2 (DM2). Myotonic dystrophy is typically inherited, following an autosomal dominant inheritance pattern, and it generally worsens with each generation. An autosomal dominant inheritance means only one copy of the mutated gene needs to be present in each cell. Children of an affected parent have a 50% chance of inheriting the mutant gene. A type of DM1 may be apparent at birth. DM2 is generally milder.

Facioscapulohumeral muscular dystrophy (FSHD) is a type of muscular dystrophy. FSHD tends to sequentially weaken the muscles of the face, those that position the scapula, and those overlying the humerus bone of the upper arm. These areas can be spared, and muscles of other areas usually are affected, especially those of the chest, spine, abdomen, and shin. Almost any skeletal muscle can be affected in severe disease. Abnormally positioned, or winged, scapulas are common, as is the inability to lift the foot, known as foot drop. The two sides of the body are often affected unequally. Weakness typically manifests at ages 15 - 30 years. FSHD can also cause hearing loss and blood vessel abnormalities in the back of the eye. FSHD is caused by a genetic mutation leading to deregulation of the DUX4 gene. Normally, DUX4 is expressed (i.e., turned on) in cells of the ovary and in very early human development, becoming repressed (i.e., turned off) by the time an embryo is several days old. In FSHD, DUX4 is inadequately repressed, allowing sporadic expression throughout life. Deletion of DNA in the region surrounding DUX4 is the causative mutation in 95% of cases, termed "D4Z4 contraction" and defining FSHD type 1 (FSHD1). FSHD caused by other mutations is FSHD type 2 (FSHD2). For disease to develop, also required is a 4qA allele, which is a common variation in the DNA next to DUX4. The chances of a D4Z4 contraction with a 4qA allele being passed on to a child is 50% (autosomal dominant); in 30% of cases, the mutation arose spontaneously. Mutations of FSHD cause inadequate DUX4 repression by unpacking the DNA around DUX4, making it accessible to be copied into messenger RNA (mRNA). The 4qA allele stabilizes this DUX4 mRNA, allowing it to be used for production of DUX4 protein. DUX4 protein is a modulator of hundreds of other genes, many of which are involved in muscle function.

Becker muscular dystrophy (BMD) is an X-linked recessive inherited disorder characterized by slowly progressing muscle weakness of the legs and pelvis. It is a type of dystrophinopathy. This is caused by mutations in the dystrophin gene, which encodes the protein dystrophin. Becker muscular dystrophy is related to Duchenne muscular dystrophy, in that both dystrophies result from a mutation in the dystrophin gene, but Becker has a milder course, compared to Duchenne muscular dystrophy.

Distal myopathy or distal muscular dystrophy (distal MD) is a group of rare genetic disorders that cause muscle damage and weakness, predominantly in the hands and/or feet. Mutation of many different genes can be causative. Distal myopathy usually appears between ages 40 and 60. But it can sometimes show up as early as the teenage years. A non-exhaustive list of the genes involved in distal myopathy: TIA1 (WDM), TTN, ZASP, GNE, DYSF, MYH7, VCP (P97), CRYAB, CAV3, and MATR3.

Congenital muscular dystrophies (CMDs) are autosomal recessively inherited muscle diseases, which means that two copies of the mutated gene need to be present in each cell, both parents need to be carriers of the mutated gene, and usually show no signs or symptoms. They are a group of heterogeneous disorders characterized by muscle weakness which is present at birth and the different changes on muscle biopsy that ranges from myopathic to overtly dystrophic due to the age at which the biopsy takes place. Indeed, there have been 35 genes discovered to be involved with different forms of CMD resulting from these mutations. There are different forms of CMD, often categorized by the protein changes caused by an atypical gene. A non-exhaustive list of these genes: LAMA2, COL6A1, COL6A2, COL6A3, SEPN1, POMT1, POMT2, FKRP, ISPD, CTDC2, TMEM5, POMGnT1, B3GALnT2, GMPPB, B3GnT1, SGK196, FKTN, MTM1, DNM2, BIN1 and TMEM5.

Emery-Dreifuss muscular dystrophy (Emery-Dreifuss MD or EDMD) is a type of muscular dystrophy, a group of heritable diseases that cause progressive impairment of muscles. EDMD affects muscles used for movement (skeletal muscles), causing atrophy, weakness, and contractures. It almost always affects the heart, causing abnormal rhythms, heart failure, or sudden cardiac death. It is rare, affecting 0.39 per 100,000 (1 per 250,000) people. EDMD is an X-linked recessive resulting of the EMD gene mutation.

Oculopharyngeal muscular dystrophy (OPMD) is a rare form of muscular dystrophy with symptoms generally starting when an individual is 40 to 50 years old. It can be autosomal dominant neuromuscular disease or autosomal recessive. The most common inheritance of OPMD is autosomal dominant. Autosomal dominant inheritance is the most common form of inheritance. Less commonly, OPMD can be inherited in an autosomal recessive pattern. The PABPN1 mutation contains a GCG trinucleotide repeat at the 5' end of the coding region, and expansion of this repeat which then leads to autosomal dominant oculopharyngeal muscular dystrophy (OPMD) disease.

Limb girdle muscular dystrophies (LGMD) are a heterogeneous group of rare muscular disorders characterized by progressive proximal muscle wasting, predominantly affecting hip and shoulders. Clinical manifestations are broad, ranging from severe forms with rapid onset and progression to slowly progressive late-onset milder forms. According to the inheritance pattern, LGMD can be classified in autosomal-dominant forms (LGMD1) and autosomal-recessive forms (LGMD2). Letters are added consecutively allowing to classify LGMD according to when individual genes were identified. LGMD1 are usually adult-onset mild forms of the disease. Among them, LGMD1A is caused by mutations in the MYOT gene encoding myotilin, a Z-disk associated alpha-actinin-binding protein involved in the structural integrity of sarcomeres. Patients exhibit proximal and distal weakness and occasional respiratory and cardiac involvement. LGMD1B is caused by mutations in the LMNA gene, encoding the nuclear membrane protein lamin A/C, implicated in several roles including mechanical maintenance of the nuclear membrane and gene regulation. Clinical manifestations of this "laminopathy" include proximal weakness, cardiac arrhythmias, and dilated cardiomyopathy. LGM1C is caused by mutations in the CAV3 gene that encodes caveolin-3, a muscle-specific sarcolemma protein component of caveolae membranes, specialized lipid rafts involved in plasma membrane maintenance, vesicular trafficking, and signal transduction. Patients exhibit moderate proximal weakness, calf hypertrophy, and muscle cramps associated to exercise. LGMD D1 is caused by mutations in DNAJB6, a member of the "DNAJ family," molecular chaperones involved in protein folding. LGMD1E associates to mutation in the DES gene encoding desmin, a component of the intermediate filaments that provides structural support to the sarcomere. LGMD D2 is caused by mutations in the transportin 3 gene (TNPO3), a nuclear receptor for serine/arginine-rich proteins. Mutations in the RNA-processing protein HNRPDL cause LGMD D3. Recessive LGMD are more frequent forms of the disease.

Among them LGMD R1 is the most prevalent LGMD worldwide accounting for up to 30% cases and is caused by mutations in the CAPN3 gene encoding calpain 3, a Ca2+-dependent non-lysosomal cysteine protease implicated in sarcomere organization and maintenance in mature muscle fibers. Most of the CAPN3 mutations result in autolysis and impaired proteolytic activity of calpain 3. Pathological features of the disease are progressive weakness and atrophy of the shoulder and pelvic girdle musculature, raised levels of serum CK and wasting and regeneration of muscle fibers in biopsy.

Recessive mutations in the DYSF gene encoding dysferlin cause LGMD R2. Dysferlin is a ubiquitous transmembrane protein implicated in Ca2+-dependent resealing of the sarcolemma after injury. Dystrophy-causing dysferlin mutations produce a drastic deficiency or complete absence of the protein; however, reduced dysferlin expression can also be observed in other muscular dystrophies secondary to mutations in other related genes. LGMDR2 account 15-25% of the LGMD2 cases and presents with slowly progressive proximal weakness that usually begin in the first or second decade of life and eventually lead to wheelchair dependence. Very highly elevated CK serum levels, nuclei internalization, muscle fibers necrosis and regeneration, and inflammatory infiltrates are typically observed. Other typical form of "dysferlinopathy" is Miyoshi myopathy, which is an adult-onset, more distal form that mainly affects posterior muscles of legs.

LGMD R3-R6 are referred as "sarcoglycanopathies" and are caused for loss of function mutations in the genes encoding alpha, beta, gamma, or delta-sarcoglycans, respectively. These are transmembrane proteins members of the sarcoglycan-complex, critical component of the DGC implicated in connecting cytoskeleton to the extracellular matrix providing mechanical stability to the skeletal muscle. Sarcoglycanopathies have a childhood onset and typically manifest with rapid or slowly progressive proximal weakness involving both, cardiac and respiratory functions.

The "dystroglycanopathies" group a number of recessive LGMD (R9, R11, R13, R14, R15, R16) linked to mutations in six genes implicated in the glycosylation of alpha-dystroglycan, critical component of the DGC: fukutin-related-protein gene (FKRP) linked to LGMDR9; O-mannosyl-transferase 1 gene (POMT1) linked to LGMDR11; fukutin gene (FKTN) associated to LGMDR13; O-mannosyl-transferase 2 gene (POMT2) linked to LGMDR14; protein O-linked-mannose beta-1,2-N-acetylglucosaminyltransferase 1 (POMGnT1) associated to LGMDR15 and the dystroglycan gene itself (DAG1) linked to LGMDR16. Abnormal glycosylation of alpha-dystroglycan inhibits its binding to extracellular matrix proteins, impairing skeletal muscle stability. Dystroglycanopathy-causing mutations produce a wide spectrum of dystrophic phenotypes ranging from mild forms such as those observed in LGMD to more severe congenital muscular dystrophies (see below). LGMDR19 is caused by mutations in the GDP-mannose pyrophosphorylase B gene (GMPPB), which catalyzes the conversion of mannose-1-phosphate and GTP to GDP-mannose, consequently causing hypoglycosylation of a-dystroglycan. LGMDR19 patients exhibit dystrophy with intellectual disabilities. Mutations in the isoprenoid synthase domain containing gene (ISPD) also impair dystroglycan glycosylation leading to LGMDR20 and also to the severe Walker Warburg syndrome. LGMDR10 is caused by mutations in the gene encoding the giant sarcomeric protein titin (TTN), mainly clustered in the C-terminal M-line-linked region of the protein, interfering with its structure and function. Mutations in the anoctamin 5 gene (ANO5) cause LGMDR12, an adulthood-onset disease that presents with asymmetric muscle weakness, pain after exercise and elevated CK levels. LGMDR17, an early-onset non-progressive form of LGMD is caused by mutations in the gene encoding plectin (PLEC1), a ubiquitously expressed protein linking actin microfilaments, microtubules, and intermediate filaments. LGMDR18 is caused by mutations in the transport protein particle complex 11 gene (TRAPPC11), a transport protein involved in anterograde membrane trafficking from the endoplasmic reticulum (ER) to the ER-to-Golgi; this form shows childhood onset ataxia, and intellectual disabilities. LGMD2W caused by mutations in the LIM and senescent cell antigen-like-containing domain protein 2 gene (LIMS2/PINCH2), that regulates cell shape and migration, presents as a childhood-onset LGMD2 with calf and tongue hypertrophy and severe quadriparesis. Mutations in the Popeye-domain-containing 1 gene (POPDC1) alter membrane trafficking and produce LGMD2X, which associates with atrio-ventricular conduction blockage. Mutations in the torsinA-interacting protein 1 gene (TOR1AIP1) encoding the lamina-associated polypeptide 1B (LAP1B) cause LGMD2Y. LGMDR21 is caused by mutations in the POGLUT1 gene encoding O-glucosyltransferase 1, an enzyme involved in Notch-posttranslational modification and function, impairing muscle regeneration mediated by Notch. Patients exhibit typical features of LGMD2 and show reduced glycosylation of a-dystroglycan. LGMDR7 and LGMDR8 are the rarest forms of LGMD2. LGMDR7 is caused by mutations in the TCAP gene that encodes telethonin, a titin-interacting protein that links titin to other Z-disk proteins supporting sarcomere assembly and muscle stretching. Clinical manifestations include adolescence-onset limb girdle weakness and cardiomyopathy susceptibility. LGMDH is a late-onset disease characterized by proximal weakness and atrophy caused by mutations in the TRIM32 gene encoding a ubiquitous E3 ubiquitin ligase involved in proteasome degradation of multiple targets, including actin.According to the invention, gene therapy is defined by an administration of a gene expression construct comprising an expression cassette containing a transgene encoding a therapeutic gene selected from micro-dystrophin protein, CALP3 protein, DYSF protein, SGCA protein, SGCG protein and FKRP protein.

The terms "gene expression construct" as used herein refers to a construction comprising an expression cassette.

The terms "expression cassette" as used herein includes a transgene of interest encoding a polypeptide to be expressed and polynucleotide sequences controlling its expression such as a promoter and a polyadenylation signal. Such cassette may also comprise an intron which is defined as a non-coding nucleic acid sequence which can modify gene expression (e.g., enhancing expression properties).

The term "polynucleotide(s)" as used herein generally refers to a plurality, two or more, of "nucleotide(s)", i.e., any polyribonucleotide or polydeoxyribonucleotide, which may be unmodified RNA or DNA or modified RNA or DNA. Polynucleotide(s) include, without limitation, single- and double-stranded DNA, DNA that is a mixture of single- and double-stranded regions or single- and triple stranded regions, single- and double-stranded RNA, and RNA that is a mixture of single and double-stranded regions, hybrid molecules comprising DNA and RNA that can be single-stranded or, more typically, double-stranded, or triple-stranded regions, or a mixture of single- and double-stranded regions. As used herein, the term "polynucleotide(s)" also includes DNAs or RNAs as described above that contain one or more modified bases. Thus, DNAs or RNAs with backbones modified for stability or for other reasons are "polynucleotide(s)" as the term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term "polynucleotide(s)" as used herein embraces such chemically, enzymatically, or metabolically modified forms of polynucleotides, as well as the chemical forms of DNA and RNA characteristic of viruses and cells, including, for example, simple and complex cells. "Polynucleotide(s)" also embraces short polynucleotides often referred to as oligonucleotide(s).

The term "polypeptide(s)" as used herein refers to any peptide or protein comprising two or more amino acids joined to each other by peptide bonds or modified peptide bonds. "Polypeptide(s)" refers to both short chains, commonly referred to as peptides, oligopeptides, and oligomers and to longer chains generally referred to as proteins. Polypeptides can contain amino acids other than the gene encoded amino acids. "Polypeptide(s)" include those modified either by natural processes, such as processing and other post-translational modifications.

The term "transgene" refers to a gene whose nucleic acid sequence is non-naturally present in the genome of a subject undergoing one of the diseases cited above.

In another particular embodiment, the transgene sequence is optimized.

Sequence optimization may include a number of changes in a nucleic acid sequence, including codon optimization, increase of GC content, decrease of the number of CpG islands, decrease of the number of alternative open reading frames (ARFs) and/or decrease of the number of splice donor and splice acceptor sites.

Because of the degeneracy of the genetic code, different nucleic acid molecules may encode the same protein. It is also well known that the genetic codes of different organisms are often biased towards using one of the several codons that encode the same amino acid over the others. Through codon optimization, changes are introduced in a polynucleotide sequence that take advantage of the codon bias existing in a given cellular context so that the resulting codon optimized polynucleotide sequence is more likely to be expressed in such given cellular context at a relatively high level compared to the non-codon optimized sequence.

In a preferred embodiment of the invention, such sequence optimized polynucleotide sequence encoding micro-dystrophin protein, CALP3 protein, DYSF protein, SGCA protein, SGCG protein or FKRP protein, is codon-optimized to improve its expression in human cells compared to non-codon optimized polynucleotide sequences coding for the same protein, for example by taking advantage of the human specific codon usage bias.

In a particular embodiment, the optimized coding sequence is codon optimized, and/or has an increased GC content and/or has a decreased number of alternative open reading frames, and/or has a decreased number of splice donor and/or splice acceptor sites, as compared to the wild-type coding sequence.

In a particular embodiment, the nucleic acid sequence of the transgene encoding the micro-dystrophin protein, CALP3 protein, DYSF protein, SGCA protein, optimized SGCA protein, SGCG protein or FKRP protein is at least 80% identical, preferably at least 81%, 82%, 83%, 84%, 85%, 86%, 87%, 88%, 89%, 90%, 91%, 92% 93%, 94%, 95%, 96% 97%, 98% or 99% identical to the wild-type human sequence.

In a preferred embodiment, the nucleic acid sequence of the transgene encoding the micro-dystrophin protein, SGCA protein, optimized SGCA protein or FKRP is at least 80% identical, preferably at least 81%, 82%, 83%, 84%, 85%, 86%, 87%, 88%, 89%, 90%, 91%, 92% 93%, 94%, 95%, 96% 97%, 98% or 99% identical to the sequence of SEQ ID NO: 1, SEQ ID NO: 2, SEQ ID NO: 3 or SEQ ID NO: 4 respectively, more preferably consists of the sequence of SEQ ID NO: 1, SEQ ID NO: 2, SEQ ID NO: 3 or SEQ ID NO: 4.

"Identity", as used herein, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in the following references (Computational Molecular Biology, Lesk A.M., ed., Oxford University Press, New York, 1988; Biocomputing: Informatics and genome Projects, Smith D.W., ed., Academic Press, New York. 1993; Computer Analysis of sequence Data, Part I, Griffin A.M., and Griffin H.G., eds., Humana Press. New jersey, 1994; sequence Analysis in Molecular Biology, von Heinje G., Academic Press, 1987; and sequence Analysis Primer, Gribskov M. and Devereux J., eds., M Stockton Press, New York, 1991; and Carillo H., and Lipman D., SIAM J. Applied Math., 48:1073 (1998)). Methods to determine identity are designed to give the largest match between the sequences tested. Moreover, methods to determine identity are codified in publicly available computer programs. Computer program methods to determine identity between two sequences include, but are not limited to, the GCG program package (Devereux J. et al., Nucleic Acids Research 12(1): 387 (1984)), BLASTP, BLASTN, and FASTA (Altschul S.F. et al., J. Molec. Biol. 215: 403-410 (1990)). The BLAST X program is publicly available from NCBI and other sources (BLAST Manual, Altschul S. et al., NCBI NLM NUH Bethesda, MD 20894; Altschul S. et al., J. Mol Biol. 215: 403-410 (1990)).

The term "variant(s)" as used herein, is a polynucleotide or polypeptide that differs from a reference polynucleotide or polypeptide respectively but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and/or truncations in the polypeptide encoded by the reference sequence, as discussed below. A typical variant of a polypeptide differs in amino acid sequence from another reference polypeptide.

Generally, differences are limited so that the sequences of the reference polypeptide and the variant are closely similar overall and, in many regions, identical. A variant and reference polypeptide may differ in amino acid sequence by one or more substitutions, additions, and/or deletions in any combination.

The present invention also includes variants of each of the polypeptides of the invention, that is polypeptides that vary from the references by conservative amino acid substitutions, whereby a residue is substituted by another with like characteristics. Typical conservative amino acid substitutions are among Ala, Val, Leu, and Ile; among Ser and Thr; among the acidic residues, Asp and Glu; among Asn and Gln; and among the basic residues Lys and Arg; or aromatic residues Phe and Tyr. Such conservative mutations include mutations that switch one amino acid for another within one of the following groups:
1. Small aliphatic, nonpolar, or slightly polar residues: Ala, Ser, Thr, Pro and Gly;
2. Polar, negatively charged residues and their amides: Asp, Asn, Glu and Gin;
3. Polar, positively charged residues: His, Arg and Lys;
4. Large aliphatic, nonpolar residues: Met, Leu, Ile, Val and Cys; and
5. Aromatic residues: Phe, Tyr, and Trp.

Particularly preferred are variants in which several, e.g., 5-10, 1-5, 1-3, 1-2 or 1 amino acids are substituted, deleted, or added in any combination. A variant of a polynucleotide or polypeptide may be naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to a person skilled in the art.

As mentioned above, in addition to the GC content and/or number of ARFs, sequence optimization may also comprise a decrease in the number of CpG islands in the sequence and/or a decrease in the number of splice donor and acceptor sites.

Of course, as is well known to those skilled in the art, sequence optimization is a balance between all these parameters, meaning that a sequence may be considered optimized if at least one of the above parameters is improved while one or more of the other parameters is not, as long as the optimized sequence leads to an improvement of the transgene, such as an improved expression and/or a decreased immune response to the transgene in *vivo.*

According to the invention, the transgene is more preferably encoding a microdystrophin protein consisting of the sequence of SEQ ID NO: 1 or a functional variant thereof having a polynucleotide sequence that is at least 80% identical to SEQ ID NO: 1.

The microdystrophin is a shortened version of wild-type dystrophin that does not include one or more spectrin homologous repeats and / or one or more rich proline hinges. In a particular embodiment, the transgene sequence encoding the microdystrophin protein, in particular the human microdystrophin protein, is optimized.

The Applicant has demonstrated in DMD dystrophic muscle the accumulation of cholesterol in the endo-lysosomal system, which was associated by lysosomal damage. Lysosomal damage is often associated by compensatory upregulation of lysosomal biogenesis. Indeed, in the dystrophic muscle in DMD the upregulation of TFEB was detected. The rational of the choice of Trehalose was therefore of the overactivation of this (TFEB-mediated lysosomal biogenesis) compensatory mechanism in the dystrophic muscle. In the examples the combined therapy in vivo in the mdx mouse model is demonstrated.

µ-dystrophin (micro-dystrophin) only was very efficient in the reduction of muscle fibrosis and the levels of circulating biomarkers. In contrast, µ-dystrophin was relatively inefficient in the reduction of lysosomal damage, as demonstrated by the Galectin-3 puncta assay. This and previous data suggest that complete reversion of lysosomal damage by AAV-pdys may require a very high viral dose, while a suboptimal dose AAV-µdys resulting in an incomplete reversion of the lysosomal damage. By contrast, Trehalose was not very efficient in reducing fibrosis, but better than AAV-µ-dys for the correction of lysosomal damage. The combination of Trehalose and AAV-pdys has shown better improvement in all tested parameters.

Thus, said carbohydrate of the present invention is preferably used as an activator of lysosomal biogenesis combined with gene therapy.

In an embodiment, said gene expression construct is comprised in a vector.

In a preferred embodiment, said vector is a plasmid vector or a viral vector.

In an advantageous embodiment, said viral vector is a recombinant adeno-associated virus (rAAV) vector or a lentivirus, preferably a recombinant adeno-associated virus (rAAV). Indeed, most of gene therapy approaches utilize adeno-associated viruses (AAVs) and lentiviruses for performing nucleic acid insertions, in *vivo* and ex *vivo,* respectively. In more than 75% of gene therapy clinical trials, a viral vector is the vehicle.

Adeno-associated virus (AAV) is the most common and powerful vector, derived from adeno-associated human parvovirus. This small virus has attracted the attention of the gene therapy field because of its low risk (not pathogenic for human and no integrative genome), the remarkable ability of recombinant AAV (rAAV) vectors to transduce with high efficiency dividing and non-dividing cells in a large variety of tissues, long-term transgene expression after injection and specific tissue tropism.

Adeno-associated virus (AAV) is a small non-enveloped DNA virus composed by an icosahedral capsid that contains a 4.7 kb linear single-stranded genome. AAV genome codes for non-structural proteins (Rep78, 68, 52 and 40), capsid proteins (VP1, VP2, VP3) and the assembly-activating protein (AAP).

AAV genome is structurally characterized by about 145-bp Inverted Terminal Repeats (ITRs) that flank two open reading frames (ORFs). The first 125 nucleotides of the ITR constitute a palindrome, which folds upon itself to maximize base pairing and forms a T-shaped hairpin structure. The other 20 bases, called the D sequence, remain unpaired. The ITRs are important cis-active sequences in the biology of AAV.

In practice, the nucleic acid sequence of interest, usually placed under the control of regulatory sequences allowing its expression, replaces the viral genome between the ITR sequences.

In a preferred embodiment, in particular in a variant wherein the gene expression construct is a single-stranded AAV genome (which is not self-complementary as explained above), the gene expression construct has a size comprised between 2100 and 5000 nucleotides, in particular between 2700 and 4900 nucleotides, more particularly between 3200 and 4700 nucleotides. In a particular embodiment, the size of the gene expression construct is of about 3200 nucleotides, about 3300 nucleotides, about 3400 nucleotides, about 3500 nucleotides, about 3600 nucleotides, about 3700 nucleotides, about 3800 nucleotides, about 3900 nucleotides, about 4000 nucleotides, about 4100 nucleotides, or about 4200 nucleotides.

According to the present invention, the term "about", when referring to a numerical value, means plus or minus 5% of this numerical value.

Recombinant AAVs may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus. Desirable AAV elements for assembly into vectors include the cap proteins, including the vp1, vp2, vp3 and hypervariable regions, the rep proteins, including rep 78, rep 68, rep 52, and rep 40, and the sequences encoding these proteins. These elements may be readily used in a variety of vector systems and host cells.

In the present invention, the capsid of the AAV vector may be derived from a naturally or non-naturally occurring serotype. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid, a recombinant AAV capsid, or a "humanized" AAV capsid. In another embodiment, the capsid is a modified capsid. In the context of the present invention, a "modified capsid" may be a chimeric capsid or capsid comprising one or more variant VP capsid proteins derived from one or more wild-type AAV VP capsid proteins.

In a particular embodiment, said rAAV vector has an AAV1, AAV2, AAV3, AAV4, AAV5, AAV6, AAV7, AAV8, AAV9 AAV9.P1, AAVMYO2, AAVMMYO3, AAV13, AAVrh10, AAVrh74, AAV9rh74, AAV9rh74-HB-P1, AAV9rh74-K-P1, AAVrh74.9, AAVanc110, AAVanc.110_9VR, AAV9_anc.110VR or LICA capsid, preferably AAV9, AAV9.rh74 or LICA.

The administration of the gene therapy treatment is, for example but not limited to, intradermal, intramuscular, intraperitoneal, intravenous, intrathecal, subcutaneous, intranasal, epidural, or oral route. In a preferred embodiment, the administration is via the intravenous or intramuscular route. The gene therapy treatment may be administered together with other biologically active agents. In a specific embodiment, it may be desirable to administer the gene therapy treatment directly to the area in need of treatment, e.g., the liver or the central nervous system. This may be achieved, for example, by means of an implant, said implant being of a porous, nonporous, or gelatinous material, including membranes, such as sialastic membranes, or fibers.
In case of a gene therapy treatment comprising administering a viral vector, such as an AAV vector, to the subject, typical doses of the vector are of at least 1×10⁸ vector genomes per kilogram bodyweight (vg/kg), such as at least 1×10⁹ vg/kg, at least 1×10¹⁰ vg/kg, at least 1×10¹¹ vg/kg, at least 1×10¹² vg/kg, at least 1×10¹³ vg/kg or at least 1×10¹⁴ vg/kg. By combining gene therapy with the carbohydrate according to this invention, the doses of the gene therapy treatment may be decreased compared to the typical doses.

In another embodiment, another aspect of the invention relates to a vector comprising the gene expression construct according to the invention.

In another preferred embodiment, the invention provides a pharmaceutical composition comprising a vector comprising the gene expression construct for use as a medicament according to preceding aspects of the invention such as previously mentioned.

The composition according to the present invention is advantageously used as a combination product for simultaneous, separate or time spread use.

In a preferred embodiment, the first active agent is the carbohydrate according to the invention and the second active agent is the vector comprising the gene expression construct according to the invention, are used as combination product for simultaneous, separate or time spread use.

In a more preferred embodiment, another aspect of the invention relates to the use of a first active agent comprising the carbohydrate according to the invention and a second active agent comprising the vector comprising gene expression according to the present invention, for the manufacture of a medicament for the treatment and/or the prevention of lysosomal damage.

In an even more preferred embodiment, another aspect of the invention relates to the use of a first active agent comprising the carbohydrate according to the invention and a second active agent comprising the vector comprising the gene expression according to the present invention, for the manufacture of a medicament for the treatment and/or the prevention of hereditary myopathy.

In an embodiment, said trehalose is administered at t = 0, and said gene expression construct is administered at t = 7 days for a continuous administration of trehalose up to 25, 24, 23, 22, 21, 20, 19, 18, 17, 16, 15, 14, 13, 12, 11, 10, 9, 8, 7, 6, 5, 4, 3, 2 or 1 years.

In another embodiment, said trehalose is continuously administered to the subject at least during one, two, tree, four, five, six, seven, eight, nine, ten, or eleven months after administration of a vector.

In another embodiment, said trehalose is administered orally and said gene construct is administered intravenously (IV) or intramuscularly (IM), preferably intravenously.

The composition according to the invention can be administered orally, in one or more identical or different formulations. It is presented in any galenic form normally used for oral administration and in particular in the form of capsule, tablet, capsule, soft capsule, dragee, sachet, tube, bottle, chewing gum, bead, emulsion, suspension, liquid, solution, ampoule, drink, syrup, powder, solid, soft gel, semi-solid.

Advantageously, the said composition is formulated as a tablet, capsule, soft gel, semi-solid, solid, liquid or powder.

An IV injection is the fastest way to inject a medication and involves using a syringe to inject a medication, here the gene construct, directly into a vein.

IM injections are given deep into a muscle where the medication, here the gene construct, is then absorbed quickly by surrounding blood vessels.

The dose of said carbohydrate, that will be effective for lysosomal correction improvement and clinical benefit of treated patients triggered by a gene therapy treatment, may also be determined by standard clinical techniques. Indeed, the dose of carbohydrate may be determined by investigation in muscle biopsies of the activation of Transcription Factor EB (TFEB) that is determined by nuclear translocation or of the reduction of Galectin-3 signal in treated animal models or humans. In addition, in *vivo* and/or *in vitro* assays may optionally be employed to help predict optimal dosage ranges; myoblast, myotubes, or organoid culture, derived from primary cells, immortalized myoblast cell lines, or iPS models can be used for such predictions. The dose to be employed may also depend on the seriousness of genetic disease (involving the use of an important dose of gene therapy treatment, leading to a higher risk to to increase viral dose and the associated risk), and should be decided according to the judgment of the practitioner and each patient's circumstances. One skilled in the art can readily determine, based on its knowledge in this field, the dosage range required based on these factors and others. In general, the doctor may also determine the appropriate dosage depending on weight and any other factors specific to the subject to be treated.

The concentration of trehalose is preferably comprised between 0.1% and 10% by weight of total water volume (g/100 ml water), preferably 1% and 5%, more preferably 2%.

### EXAMPLES

### Example 1: Materials & methods

**In vivo mice experiments:** All animals were handled according to French and European guidelines for human care and use of experimental animals. Experiments on mice were approved by the ethical committee n°C2AE-51 of Evry and the regulatory affairs of the French Ministry of Research (MESRI) under the APAFIS number 35896 (DAP 2022-004-C). Mice were housed in a SPF barrier facility with 12-h light/dark cycle and provided with food and water ad libitum. In this study, only male mice were used. C57BL/6J (WT) and mdx-4Cv (B6Ros.Cg-Dmdmdx-4Cv/J) mice were obtained from Charles River laboratories.

**Gene therapy experiment in a mouse model for Duchenne muscular dystrophy:** A Full material and method chapter concerning this investigation can be found in Bourg et al, 2022 . Briefly: The Opt-human MD1 (OH-MD1-µDYS) construct is our optimized version of the microdystrophin that was developed by the laboratories of Jeffrey Chamberlain (H2mDys) and George Dickson (MD1µDYS) and used (the dog version) in Genethons' preclinical investigation in the Golden retriever muscular dystrophy (GRMD) dog model. It is a spectrin-like repeat 4 to 23 deleted, C-terminal truncated microdystrophin (µdysΔ4-23ΔCT under the transcriptional control of the artificial Spc5.12 promoter). To obtain this version of microdystrophin the MD1µDYS was modified further, including additional codon-optimization (for human), removal of CpG methylation sites, removal of antisense ORFs and of sense ORFs larger than 100bp. The modified vector was designated OH-MD1-µDYS.

**Generation and titration of recombinant AAV vector:** HEK293-T cells, cultured in suspension, were transfected with the three plasmids coding for the adenovirus helper proteins, the AAV Rep and Cap proteins, and the ITR-flanked transgene expression cassette. Three days after the transfection, cells were harvested, chemically lysed, treated with benzonase (Merck-Millipore, Darmstadt, Germany), and filtered. Viral capsids were purified by affinity chromatography, formulated in sterile PBS, and the vector stock was stored at -80°C.

**Mice treatment:** 3-weeks old mdx male mice were used for the experience. Trehalose (Sigma T9449) was diluted in drinking water at a 2% concentration (2g/100mL) and drinking water was changed twice a week from beginning of treatment until sacrifice at four weeks. For AAV treated mice, AAV9 OH-MD1-µDYS vector was diluted in physiological serum at a titer of 7 × 1012 viral genomes per kg (vg/kg) and injected intravenously at day 7.

**Evaluation of muscle force:** The motor capacity of the mice was evaluated using the escape and test. The procedure follows the recommendations of TREAT NMD SOP for the DMD animal models [Luca, A.; Nagaraju, K.; et al. Update on Standard Operating Procedures in Preclinical Research for DMD and SMA Report of TREAT-NMD Alliance Workshop, Schiphol Airport, 26 April 2015, The Netherlands. J. Neuromuscul. Dis. 2018, 5, 29-34. https://doi.org/10.3233/JND-170288.]. Briefly, the mouse is placed on a platform inside a 30 cm tube, tail-connected to a force measurement devise, to record the escape force. A mean of the five highest scores out of max 15, during max 5 min (the first to be reached) is normalized to the weight of the mouse. The escape test is performed in only one session (i.e., the indicated number of repetitions in one single training session), since mice are unwilling to perform the escape test a second time.

**mCK Quantification:** Blood samples were collected by retro-orbital bleeding and quickly centrifuged for 10 min at 8000 rpm. Sera were harvested and further centrifuged to completely remove cell contaminants. The sera were finally stored at -80°C until measurement. Ten µL of mouse serum was used to colorimetrically measure creatine phosphokinase concentration by FUJI DRI-CHEM nx500 system (DMV Imaging).

**Histological Analyses:** Skeletal muscles were dissected out and frozen in isopentane cooled in liquid nitrogen. Muscles were fixed with gum and Optimal Cutting Temperature (OCT) Tissue Tek on a cork base and stored at -80°C. Transverse cryosections (8µm thickness) were prepared from frozen tissues, air dried, and stored at -80°C. Sampling for biomolecular analysis was also performed at the same time with trim cuts of the frozen tissues. H&E staining was done according to standard procedures. Red Sirius staining was used to detect fibrosis on muscle sections. Briefly, Slides were treated with Acetone for 1 hour, fixed in formol for 5 minutes and then incubated in Bouin's solution for 10 min. Slides are then rinsed in water twice and stained in a Red Sirius solution (Sigma 365548) (0,1% in picric acid) for 1 hour, washed in water and dehydrated in ethanol (70% 1 min, 95% 1 min, 100% for 2 min) and xylene (2 wash for 1 min). Slides are mounted in mounting media and air dried.

Images were digitalized using an Axioscan Z1 slide scanner (Zeiss, Germany) under a Zeiss Plan-Apochromat 10X/0.45 M27 dry objectif (ZEISS, Germany). Tile scan images were reconstructed with ZEN software (ZEISS, Germany). Quantification of fibrosis positive area was done using QuPath software.

**RT-qPCR:** Frozen tissue sections were treated with Nucleozol (Macherey-Nagel, Ref: 740404.200) and then grinded with the Fast Prep Bead Mill 2000 (FisherbrandTM) at 6m/s for 40 seconds. Total RNA was extracted with nucleoMag B-Beads (Macherey-Nagel 744503.12) using an automated system (IDEAL 32 - ID solutions), and then purified with TURBO DNAse (ThermoFisher, AM1907). The RevertAid H Minus Reverse Transcriptase kit (ThermoFisher, Ref.EP0451) was used to produce cDNA, both random hexamers and oligodT were used (1:10 ratio). One µg of purified RNA is used for cDNA synthesis at the following settings: 10 min/25°C, 1h15/42°C, 10 min/70°C. At the end, cDNA is diluted (1/8,5) and used for qPCR experiments.

SYBR green assay was used for the quantification of the µ-dystrophin transgene expression using the forward primer (5'- AGCAAGAGCACAACAATTTGGT -3') and the reverse primer (5'- CCCTGTTCGTCCCGTATCATAA-3'). The following program was run using a LightCycler490 (Roche): Denaturation 95°C/10 min, 40 cycles (95°C/15sec - 60°C 1 min - 2°C/sec). Expression was normalized to rplp0 expression (forward primer: 5' AGCCCAGAACACTGGTCTC-3'; Reverse primer: 5'-ACTCAGGATTTCAATGGTGCC -3') using the 2-DeltaCt Livak method.

**Galectin-3 immunofluorescence:** Slides were dried out at room temperature for 10 min. PAP-Pen is used to draw circles around muscle sections, and slides were re-hydrated in PBS for 5 minutes. Slides were then fixed in ice cold 4% paraformaldehyde (PFA) for 3 min and washed 2 times in PBS after. Slides were then permeabilized in an ice-cold methanol gradient (10 min 70%; 10 min 95%, 20 min 100%). After3 washing with PBS (5min), a solution of Goat serum (10%) was used for blocking at room temperature for 30 min. A Mouse-on-Mouse solution (Invitrogen R37621) is then used for blocking endogenous binding to mouse IgG (30 min RT). Slides were incubated then in a primary antibodies solution (LAMP2, Rat monoclonal - 1/200 abcam ab13524; LGALS3, Mouse Monoclonal IgG1 - 1/50 , SantaCruz sc32790) diluted in 1% Goat Serum. Slides were washed in TTBS solution 3 times for 5 minutes then incubated in a secondary antibody solution diluted in 1% Goat Serum for 2h at 4°C (Goat Anti-Rat Alexa488, Invitrogen A1 1006 1/200; Goat Anti-Mouse IgG1 Alexa 594 1/200). Finally, slides are washed in PBS solution 3 times for 5 minutes, rinsed in milliQ water and mounted with a DAPI-Fluoromount-G mounting media (Southern Biotech ref. 0100-20) and dried out. Images were acquired using a spectral LEICA TCS SP8 confocal microscope (Leica, Germany) operating under LASX Software (Leica, Germany) using a HC PL APO CS2 20X 0.75 NA dry objective. Acquired images are cropped with ImageJ for final figures.

## Claims

1. A composition comprising at least one carbohydrate for use in the prevention and/or treatment of lysosomal damage, wherein said composition is combined with gene therapy.

2. The composition for use according to claim 1, wherein said carbohydrate is a disaccharide.

3. The composition for use according to claim 1 or 2, wherein said carbohydrate is selected from trehalose, lactulose and/or melibiose, preferably trehalose.

4. The composition for use according to any one of preceding claims, for use in the prevention and/or treatment of hereditary myopathy.

5. The composition for use according to claim 4, wherein hereditary myopathy is selected from congenital myopathies, autophagic vacuolar myopathy, muscular dystrophy, and wherein:
- autophagic vacuolar myopathy is preferably inclusion body myositis, and toxic myopathies; and
- muscular dystrophy is preferably DMD, myotonic dystrophy, Facioscapulohumeral muscular dystrophy (FSHD), Becker muscular dystrophy (BMD), distal Muscular Dystrophy, congenital muscular dystrophy, Emery-Dreifuss Muscular Dystrophy, Oculopharyngeal Muscular Dystrophy (OPMD), Limb girdle muscular dystrophies (LGMD) advantageously selected from Calpain-3-related limb-girdle muscular dystrophy R1 (LGMDR1), Dysferlin related limb girdle muscular dystrophy R2 (LGMDR2), Alpha sarcoglycan related limb girdle muscular dystrophy R3 (LGMDR3), Gamma sarcoglycan related limb girdle muscular dystrophy R5 (LGMDR5), FKRP related limb girdle muscular dystrophy R9 (LGMDR9), Dysferlin related limb girdle muscular dystrophy R2 type 2B (LGMD2B/R2), X-linked myotubular myopathy, BIN1-related centronuclear myopathy and DNM2-related centronuclear myopathy.

6. The composition for use according to claims 1 to 5, wherein the gene therapy is an administration of a gene expression construct comprising an expression cassette containing a transgene encoding a therapeutic gene selected from micro-dystrophin protein, CALP3 protein, DYSF protein, SGCA protein, SGCG protein and FKRP protein, MTM1 protein, BIN1 protein, DNM2 protein, GNE protein, Caveolin-3 protein, VCP protein, LARGE protein, and LMNA protein.

7. The composition for use according to any one of preceding claims, wherein the gene therapy is an administration of a gene expression construct comprising an expression cassette containing a transgene encoding a microdystrophin protein consisting of the sequence of SEQ ID NO: 1 or a functional variant thereof having a polynucleotide sequence that is at least 80% identical to SEQ ID NO: 1.

8. The composition for use according to claim 6 or 7, wherein said gene expression construct is comprised in a vector.

9. The composition for use according to claim 8, wherein said vector is a plasmid vector or a viral vector.

10. The composition for use according to claim 9, wherein said viral vector is a recombinant adeno-associated virus (rAAV) vector or a lentivirus, preferably a recombinant adeno-associated virus.

11. The composition for use according to claim 10, wherein said rAAV vector has an AAV1, AAV2, AAV3, AAV4, AAV5, AAV6, AAV7, AAV8, AAV9 AAV9.P1, AAVMYO2, AAVMMYO3, AAV13, AAVrh10, AAVrh74, AAV9rh74, AAV9rh74-HB-P1, AAV9rh74-K-P1, AAVrh74.9, AAVand 10, AAVanc.110_9VR, AAV9anc.110VR or LICA capsid, preferably AAV9, AAV9.rh74 or LICA.

12. The composition for use according to any one of preceding claims, wherein said composition is used as a combination product for simultaneous, separate or time spread use.

13. The composition for use according to claim 12, wherein said trehalose is administered orally and said gene construct is administered intravenously or intramuscularly, preferably intravenously.
